(19) Europäisches Patentamt
European Patent Office
Office européen des brevets

(11) Publication number: **0 343 798**
**A1**

# (12) EUROPEAN PATENT APPLICATION

(21) Application number: 89304359.6

(22) Date of filing: 28.04.89

(51) Int. Cl.⁴: C07C 37/52 , C07C 39/15

(30) Priority: 26.05.88 GB 8812539

(43) Date of publication of application:
29.11.89 Bulletin 89/48

(84) Designated Contracting States:
AT BE CH DE FR GB IT LI LU NL SE

(71) Applicant: IMPERIAL CHEMICAL INDUSTRIES PLC
Imperial Chemical House Millbank
London SW1P 3JF(GB)

(72) Inventor: Staniland, Philip Anthony
1 The Croft Marton
Middlesbrough Cleveland(GB)
Inventor: Candlin, John Paton
7 Rudby Lea Hutton Rudby
Yarm Cleveland(GB)

(74) Representative: Graham, John George et al
Legal Dept. Patents PO Box 6 Bessemer Road
Welwyn Garden City Hertfordshire AL7 1HD(GB)

## (54) Preparation of aromatic compounds and derivatives thereof.

(57) 4,4″-Dihydroxy-p-terphenyl, 4-hydroxybiphenyl and related compounds are prepared by condensation of cyclic diones or ketones with phenols and dehydrogenation in the presence of a catalyst (Pd-C) and base. The reactions may be carried out as a one-pot process.

EP 0 343 798 A1

## Preparation of aromatic compounds and derivatives thereof

This invention relates to a process for preparation of 4-hydroxyphenyl aromatic compounds, particularly bis(4-hydroxyphenyl) aromatic compounds, and derivatives thereof.

4,4″-Dihydroxy-p-terphenyl, and related compounds find application in manufacture of polyethers, polyetherketones, polyethersulphones, polyesters, polycarbonates and other polymers. The unsubstituted compounds are particularly useful in manufacture of high performance engineering polymers.

According to a first aspect of the present invention a process for preparation of bis(4-hydroxyphenyl) aromatic compounds and derivatives thereof of formula I

X - Ph - Ar - Ph - x    I

wherein:-

Ar is a substituted or unsubstituted aromatic group selected from 1,4- phenylene, 1,3-phenylene or a polyaromatic group containing at least two aromatic groups which are either directly linked together or are linked together by CO, $SO_2$, O, S or $CR_2$ where each R is independently hydrogen, or a $C_1$ to $C_4$-alkyl or -fluoroalkyl or phenyl;

Ph is l,4-phenylene optionally substituted with one or two substituents selected from $C_1$ to $C_4$-alkyl, halogen or hydroxy; X is OH, SH, $OR^2$ or $SR^2$ where $R^2$ is $C_1$ to $C_4$-alkyl, for example methyl or tert-butyl or is benzyl;

comprises the step of dehydrogenation of a compound of formula II

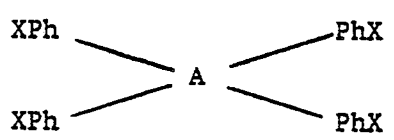

II

wherein:-

A is a saturated or partially saturated cyclic group which yields a group Ar upon removal of hydrogen.

By "directly linked together" we mean that the aromatic groups are linked by a direct link between the rings as in biphenylene and terphenylene or are linked together in a fused ring system as in naphthalene or pentalene.

The groups Ph, if substituted, are preferably substituted at the 2 or 3-positions relative to the group X.

Examples of polyaromatic groups Ar include $PhSO_2Ph$, PhCOPh, PhOPh, PhSPh, $PhCR^3{}_2Ph$ (where each $R^3$ is independently H, $CH_3$ or $C_6H_5$), and naphthylenes (1,4; 1,5; 2,6; 2,7).

The dehydrogenation may be carried out in presence of a dehydrogenation catalyst. These include platinum catalysts, for example: platinum-carrier catalysts (eg Pt-C); metallic platinum; platinum oxide etc: palladium catalysts, for example : palladium-carrier catalysts (eg Pd-C, Pd-$BaSO_4$, Pd-MgO, Pd-CaO, Pd-$Al_2O_3$); metallic palladium; palladium oxide etc: rhodium catalysts, for example: rhodium-carrier catalysts (eg Rh-C): ruthenium or rhenium catalysts. Alternative catalysts include: Raney nickel, Raney cobalt, or Raney copper; reduced nickel, cobalt or copper; nickel-carrier, cobalt-carrier or copper-carrier catalysts. Use of a platinum group metal is preferred if the substrate does not contain reactive groups.

The amount of platinum group metal dehydrogenation catalyst may lie within the range 0.001 to 0.2 moles of catalytic metal relative to the compound II but alternative proportions may be used as convenient.

Partial dehydrogenation may be achieved by thermal decomposition of the compounds of formula II in the presence of base eg NaOH.

The dehydrogenation may be performed in the presence of acid or base although neither need be employed. Use of a base is preferred for example: alkali metal hydroxide such as sodium hydroxide or potassium hydroxide, alkaline earth metal hydroxide such as magnesium hydroxide or barium hydroxide, carbonates such as sodium carbonate, acetates and phenoxides. Use of a strong base, eg sodium hydroxide, is preferred.

Acid catalysts include Lewis acid, eg aluminium chloride, ferric chloride and stannic chloride; sulphonic acids, eg 4-toluenesulphonic acid and triflic acid $CF_3SO_3H$.

A hydrogen acceptor may be employed, although this is not essential. Conventional hydrogen acceptors which may be used include sulphur, selenium, quinone and unsaturated compounds eg alkenes or alkynes. Azo, nitro, carbonyl or phenolic compounds may also be used. Use of unsaturated compounds is preferred eg $\alpha$-methylstyrene, or phenol, particularly if a useful product is formed. Thus, $\alpha$-methylstyrene yields cumene and phenol yields cyclohexanone or cyclohexanol.

The dehydrogenation is preferably carried out at temperatures of 100° to 400° C more preferably 150° to 300° C. Use of a solvent is preferred. Conventional dehydrogenation solvents may be employed. Thus, reactions in solution are generally conducted at reflux in relatively high-boiling solvents, such as cumene, p-cymene, decalin, nitrobenzene, naphthalene, quinoline or 1-methylnaphthalene. The polyglycol ethers, eg triglyme, offer the advantages of a wide range of boiling point and convenience of removal during workup by virtue of their miscibility with water. Vigorous boiling or passage of a stream of inert gas through the solution is frequently employed both to stir the mixture and to sweep hydrogen from the system as it is liberated. The hydrogen acceptor may be used as a solvent. In a preferred process the hydrogen acceptor is a further quantity of a compound of formula III

HPhX     III

eg phenol, used for the condensation reaction producing the compound of formula II. Water may be present in the dehydrogenation stage.

The formation of triaryl compounds is advantageous in that the dehydrogenation may proceed at a lower temperature than in formation of biaryl compounds for example from cyclohexanone and phenol. This may be attributable to the formation of 2 double bonds upon elimination of 2 molecules of HPhX, eg phenol, from the tetrakis compound, leaving only one double bond to be created during dehydrogenation to the aromatic product.

The compounds of formula II may be prepared by condensation of a compound of formula III with a cyclic dione. A convenient procedure, described in US 4277600 involves passage of hydrogen chloride through a mixture of these reagents.

Suitable diones include: cyclohexan-1,4-dione, cyclohexan-1,3-dione, bicyclohexyl-4,4′-dione, bicyclohexyl-3,4′-dione, propan-2,2-bis (cyclohexan-4-one), bicyclohexan [3,3,0]-2,5-dione (pentalandione) decalin-1,5-dione, decalin-1,4-dione, decalin-2,6-dione, bis(cyclohexyl)ketone-4,4′-dione and bis(cyclohexyl)-sulphone-4,4′-dione. Protected or masked diones may be used eg bis-ketals or tetraacetates of these diones.

Substituted diones may be employed. Suitable substituents include R and $CO_2R$ where A is $C_1$ to $C_4$-alkyl.

According to a second aspect of this invention a process for preparation of a 4-hydroxyphenyl aromatic compound or derivative thereof of formula IV

$(X - Ph)_n - Ar$     IV

wherein Ar, Ph and X are as previously defined and n is 1 or 2; comprises the steps of formation of compound V

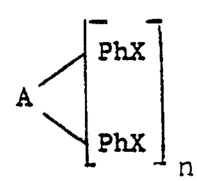

V

wherein A is as previously defined, by condensation of a compound of formula III

HPhX     III

with a cyclic mono- or di-ketone corresponding to group A and dehydrogenation of compound V, said dehydrogenation being performed without isolation of compound V.

The cyclic mono- or di-ketone may be introduced in protected or masked form.

It will be appreciated that when n = 2, compound IV is compound II hereinbefore defined.

This process finds particular application in commercial scale production of biphenyl and terphenyl compounds. For example the compounds disclosed in EP 240362 and EP 251614 may be economically prepared by this process. The process makes operation in a single reaction vessel possible and this greatly facilitates industrial exploitation of the invention.

In the condensation stage the compound III reactant is present preferably in excess for example 2 to 20, especially 5 to 10 times the proportion of keto groups. It can thus act as solvent in both stages and as a hydrogen acceptor in the dehydrogenation stage.

The condensation reaction is preferably acid-catalysed. Suitable acid catalysts include Bronsted acids (optionally in the presence of water) for example HX (where X = F, Cl, Br, I), $H_2SO_4$, $H_3PO_4$, halogenated acetic acids, sulphonic acids, eg benzene sulphonic acid, triflic acid, polymeric acids eg cationic ion exchange resins containing sulphonic acid groups (eg Amberlyst 15, (Registered Trade Mark), Nafion-H (Registered Trade Mark). Lewis acids may be employed eg $BF_3$, $AlCl_3$, $ZnCl_2$ or aluminium phenate either

alone or in combination with the above Bronsted acids. Acid forms of zeolites can be used. If water is present, at for example 1-10% w/w on the total reaction mixture, the purity of the product of the subsequent dehydrogenation appears to be unaffected. In particular concentrated hydrochloric acid may be used in place of gaseous hydrogen chloride, the water present in the hydrochloric acid and also that formed by the condensation reaction being subsequently removed by distillation.

In addition, promoters containing thiol or thioether groups eg $H_2S$, alkyl mercaptans, mercapto-amines or thiazoles can be used in conjunction with the above acids.

The compound III reactant can be any such compound not substituted in the 4-position. In preferred compounds, X is OH. The ketone reactant can be any of the above diones or corresponding mono-ketones for example.

For the dehydrogenation stage the solution of compound V, which contains unreacted compound III, is made alkaline by addition of strong base, eg NaOH. A dehydrogenation catalyst eg Pd-C, is added and the temperature raised to 160-200 °C to distil off water. The mixture is then heated under reflux until dehydrogenation is complete (eg 4 hr), then cooled and acidified. After acidification, the product compound IV may be simply filtered from the resultant mixture, optionally after addition of water to maintain the unreacted and generated phenol as a solution to facilitate filtration at a conveniently lower temperature.

Base catalysts can also be used to form compound V, eg Na or K phenate. In this event the addition of alkali before dehydrogenation is unnecessary.

The invention is further described by means of examples, but not in any limitative sense.

Example 1

The tetraphenol 1,1,4,4-tetrakis(4-hydroxyphenyl)cyclohexane 15 was prepared according to the method disclosed in Examples 1 of US 4776004.

The tetraphenol (mp 316-324 °C) was identified and shown to be at least 95% pure by $^1$H and $^{13}$C nmr.

Example 2-Preparation of 4,4″-dihydroxy-p-terphenyl

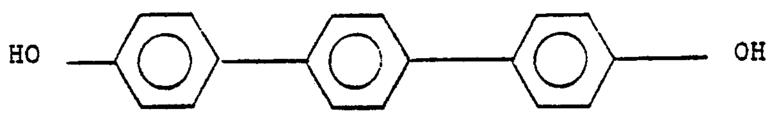

1,1,4,4-Tetrakis(4-hydroxyphenyl)cyclohexane (13.58g) was heated for 3.5 hr at 250 °C in a stainless steel autoclave with sodium hydroxide (0.82g), 5% palladium on charcoal (0.12g), α-methylstyrene (6.60g) and water (300 ml). Additional sodium hydroxide (2.0g) was added and the mixture was poured with stirring into glacial acetic acid (50 ml). The solid product was collected, washed with water and dried under vacuum at 50 °C.

The 4,4″-dihydroxy-p-terphenyl (7.88g) was characterised and shown to be 95% pure by $^1$H and $^{13}$C nmr. The $^1$H nmr spectrum contained resonances at 6.98, 7.63 and 7.73 ppm. The $^{13}$C nmr spectrum contained resonances at 115.6, 125.9, 127.1, 130.3, 138.0 and 156.9 ppm. Found C = 81.84%, H = 5.51% Calc C = 82.42%, H = 5.38%. The melting point of the product was determined in a sealed, evacuated tube and was 378.6-380.6 °C..

Example 3 'One-pot' process synthesis of 4,4″-dihydroxy-p-terphenyl

To a 1 l three-necked flask fitted with nitrogen inlet, stirrer, thermometer and long air condenser was charged 1,4- cyclohexadione (0.40 mole, 44.8g) and phenol (6.4 mole, 600g). The mixture was heated to 50 °C to form a solution and concentrated hydrochloric acid (sg = 1.18) (20 ml) was added. The mixture was then stirred at 50 °C for 7.0 hours.

Sodium hydroxide (0.75 mole, 30g) and 5% palladium on carbon (2.0g) was added and with the condenser arranged for distillation, low boiling materials were removed (ca 50 ml) until the pot temperature reached 180 °C. The mixture was then left at reflux (180 °-185 °C) for 4.0 hours, cooled to 50 °C and glacial acetic acid (60 ml) and water (100 ml) added.

The solid product was collected, washed with water (3 x 300 ml) at 50° C until the odour of phenol was no longer apparent and dried under vacuum at 100° C; yield 36.7g (35.0% yield based on l,4-cyclohexadione taking into account the catalyst (2.0g)).

The product was recrystallised from N-methyl pyrrolidone (NMP) (175 ml), heated to 150° C and filtered to remove the Pd/C catalyst. The product was collected, washed once with cold NMP (50 ml) and twice with methanol (50 ml) dried under vacuum at 120° C for 3 hrs.

The yield was 24.43g and the mp was 378° - 381° C. The infra red spectrum was identical with that of the product of Example 2.

Example 4 'One-pot' process synthesis of 4,4″-dihydroxy-p-terphenyl

To a 500ml three-necked flask fitted with nitrogen inlet, stirrer, thermometer and long air condenser was charged 1,4- cyclohexadione bis(ethylene ketal) (0.20 mole, 40.04g) and phenol (2.13 mole, 200g). The mixture was heated to 70° C to form a solution, cooled to 50° C and concentrated hydrochloric acid (10 ml) was added. The mixture was then stirred at 50° C for 6.0 hours, then allowed to stand at room temperature overnight.

Whilst stirring, sodium hydroxide pellets (0.38 mole, 15g) were added followed by 5% palladium on carbon (1.0g). The apparatus was arranged for distillation, and the reaction mixture was heated until it reached 180° C. The mixture was then left at reflux (180° C) for 4 hours 20 minutes, cooled to 50° C and glacial acetic acid (30 ml) followed by water (50 ml) were added. On further cooling to room temperature , a solid was collected, washed thrice with water and dried under vacuum at 100° C.

The product (yield 10g) was recrystallised from N-methyl pyrrolidone to give 7.10g of material with a mp of 378° C. The infra red spectrum was identical with that of the product of Example 2.

## Claims

1. A process for preparation of bis(4-hydroxyphenyl) aromatic compounds and derivatives thereof of formula I

X - Ph - Ar - Ph - X    I

wherein:-

Ar is a substituted or unsubstituted aromatic group selected from 1,4- phenylene, 1,3-phenylene or a polyaromatic group containing at least two aromatic groups which are either directly linked together or are linked together by CO, $SO_2$, O, S or $CR_2$ where each R is independently hydrogen, or a $C_1$ to $C_4$-alkyl or -fluoroalkyl or phenyl;

Ph is 1,4-phenylene optionally substituted with one or two substituents selected from $C_1$ to $C_4$-alkyl, halogen or hydroxy; X is OH, SH, $OR^2$ or $SR^2$ where $R^2$ is $C_1$ to $C_4$-alkyl or is benzyl;

comprising the step of dehydrogenation of a compound of formula II

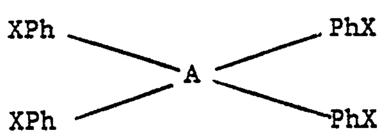

II

wherein:-

A is a saturated or partially saturated cyclic group which yields a group Ar upon removal of hydrogen.

2. A process for preparation of a 4-hydroxyphenyl aromatic compounds or derivatives thereof of formula IV

$(X - Ph)_n$ - Ar    IV

wherein:-

Ar is a substituted or unsubstituted aromatic group selected from 1,4- phenylene, 1,3-phenylene or a polyaromatic group containing at least two aromatic groups which are either directly linked together or are linked together by CO, $SO_2$, O, S or $CR_2$ where each R is independently hydrogen, or a $C_1$ to $C_4$ -alkyl or -fluoroalkyl or phenyl;

Ph is 1,4-phenylene optionally substituted with one or two substituents selected from $C_1$ to $C_4$ -alkyl,

halogen or hydroxy; X is OH, SH, $OR^2$ or $SR^2$ where $R^2$ is $C_1$ to $C_4$-alkyl or is benzyl; and n is 1 or 2;
comprising the steps of formation of compound V

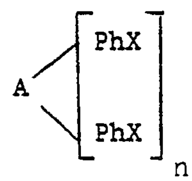

V

wherein:-
A is a saturated or partially saturated cyclic group which yields a group Ar upon removal of hydrogen;
by condensation of a compound of formula III
HPhX III
with a cyclic mono- or di-ketone corresponding to group A and dehydrogenation of compound V, said dehydrogenation being performed without isolation of compound V.

3. A process according to claim 1 or claim 2, in which the dehydrogenation step is carried out in the presence of a dehydrogenation catalyst selected from at least one platinum group metal.

4. A process according to any one of the preceding claims, in which the dehydrogenation step is carried out in the presence of a base.

5. A process according to any one of the preceding claims, in which the dehydrogenation step is carried out in the presence of a hydrogen acceptor.

6. A process according to any one of the preceding claims, in which the reaction is carried out in a solvent.

7. A process according to claim 5 and claim 6, in which the hydrogen acceptor is the solvent.

8. A process according to any one of the preceding claims, in which the dehydrogenation step is partially achieved by thermal decomposition in the presence of a base.

9. A process according to any one of the preceding claims, in which the compound of formula

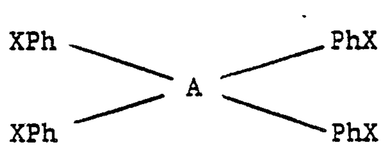

II

is prepared by the condensation of a compound of formula III
HPhX III
with a cyclic dione corresponding to group A.

10. A process according to any one of the preceding claims, in which X is OH.

European Patent Office

# EUROPEAN SEARCH REPORT

Application Number

EP 89 30 4359

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | PATENT ABSTRACTS OF JAPAN, vol. 11, no. 133 (C-418)[2580], 25th April 1987; & JP-A-61 268 640 (MITSUI TOATSU CHEM. INC.) 28-11-1986 --- | 2,5-7, 10 | C 07 C 37/52<br>C 07 C 39/15 |
| A | GB-A-1 205 794 (UNION CARBIDE) * Claims 1-3,6-7,11-12,17 * ----- | 2-4,8, 10 | |
| | | | **TECHNICAL FIELDS SEARCHED (Int. Cl.4)** |
| | | | C 07 C 37/00<br>C 07 C 39/00 |

The present search report has been drawn up for all claims

| Place of search | Date of completion of the search | Examiner |
|---|---|---|
| THE HAGUE | 01-08-1989 | KLAG M.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)